(19) Europäisches Patentamt
European Patent Office
Office européen des brevets

(11) Veröffentlichungsnummer: **0 158 174**
**B1**

# (12) EUROPÄISCHE PATENTSCHRIFT

(45) Veröffentlichungstag der Patentschrift: **01.04.87**

(51) Int. Cl.⁴: **C 11 D 1/825**, C 11 D 1/72, C 11 D 17/00, A 61 K 7/08

(21) Anmeldenummer: **85103217.7**

(22) Anmeldetag: **20.03.85**

## (54) Nichtionische, fliessfähige Perlglanzdispersionen.

(30) Priorität: **28.03.84 DE 3411328**

(43) Veröffentlichungstag der Anmeldung: **16.10.85 Patentblatt 85/42**

(45) Bekanntmachung des Hinweises auf die Patenterteilung: **01.04.87 Patentblatt 87/14**

(84) Benannte Vertragsstaaten: **BE DE FR GB IT NL**

(56) Entgegenhaltungen:
**DE - A - 3 212 420**
**GB - A - 2 121 072**

**PATENT ABSTRACTS OF JAPAN, Band 5, Nr. 136, 28. August 1981, Seite (C-69) (808);**

(73) Patentinhaber: **HOECHST AKTIENGESELLSCHAFT, Postfach 80 03 20, D-6230 Frankfurt am Main 80 (DE)**

(72) Erfinder: **Quack, Jochen Meinhard, Dr., Wilhelm-Reuter-Strasse 16, D-6239 Eppstein/Taunus (DE)**
Erfinder: **Reng, Alwin, Im Schulzehnten 22, D-6233 Kelkheim (Taunus) (DE)**
Erfinder: **Skrypzak, Werner, Eichkopfallee 33, D-6237 Liederbach (DE)**
Erfinder: **Kunz, Walter, Karl-Staib-Strasse 21, D-6234 Hattersheim am Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Zur Verbesserung des optischen Aspektes von kosmetischen Haar- und Körperreinigungsmitteln, Spülmitteln und flüssigen Wasch- und Reinigungsmitteln werden häufig Substanzen verwendet, die den genannten Produkten ein perlglanzartiges Aussehen verleihen. Zur Erzielung eines solchen Perl- bzw. Seidenglanzeffektes sind verschiedene Substanzen bekannt, beispielsweise pulverförmige Naturstoffe wie Glimmer, Fischsilber (modifizierte Fischschuppen), anorganische Materialien wie Wismutoxichlorid und Titandioxidpigmente, ferner Metallsalze von höheren Fettsäuren, Fettsäureglykolester und Fettsäurealkanolamide im Gemisch mit anderen Tensiden.

Eine in letzter Zeit häufig angewandte Technik ist ein Verfahren, bei dem ein Fettsäureglykolester allein oder in Kombination mit Fettsäurealkylolamiden verwendet wird. Da es sich bei den genannten Materialien um feste, nichtkristalline Substanzen handelt, ist bei der Herstellung von kosmetischen Wasch- und Reinigungsmitteln mit Perlglanzeffekt ein Erwärmen des Ansatzes über den Schmelzpunkt der verwendeten perlglanzgebenden Substanzen erforderlich. In diesem Falle wird nach dem Erhitzen eine homogene Schmelze erhalten, aus der nach dem Abkühlen durch Kristallisation ein Perlglanzeffekt entsteht. Dieses Verfahren besitzt jedoch den Nachteil, dass hierbei der gesamte Produktionsansatz auf eine geeignete Temperatur (je nach Perlglanzbildner ca. 60-80°C) erhitzt werden muss und erst durch exakt definierte Abkühl- und Rührbedingungen ein mehr oder minder gleichmässiger konstanter Perlglanz erzeugt wird. Hierbei ist ein relativ hoher Energie- und Zeitaufwand erforderlich.

Die Zugabe von wärme- bzw. hitzempfindlichen Substanzen wie ätherische Öle, Parfümöle und spezielle Wirkstoffe kann erst nach Beendigung des Abkühlvorganges erfolgen. Die Möglichkeit der kontinuierlichen Herstellung von kosmetischen Präparaten mit Perlglanzeffekt ist mit diesem Verfahren ebenfalls nicht gegeben.

Aufgrund der oben beschriebenen Nachteile haben sich in jüngster Zeit Verfahren etabliert, die es ermöglichen, kosmetische Haar- und Körperreinigungsmittel mit Perlglanzeffekt auch bei Raumtemperatur herzustellen. Bei diesen Verfahren werden sogenannte Perlglanzdispersionen, basierend auf verschiedenartigen Perlglanzbildnern in Kombination mit Tensiden eingesetzt. Hierbei wird zur Erzielung eines Perlglanzeffektes die perlglanzgebende Substanz in hoher Konzentration oberhalb der Schmelztemperatur in das Tensid, das ebenfalls in hoher Konzentration vorliegt, eingearbeitet. Durch exakte Abkühl- und Rührbedingungen werden hierbei Perlglanzkristalle mit einer definierten Teilchengrössenverteilung gebildet.

Die derzeit verwendeten Substanzen zur Herstellung dieser Perlglanzdispersionen sind Fettsäuremono- und/oder Polyglykolester, die alleine oder in Kombination mit Alkanolamiden in anionische Tenside, wie z.B. Alkylsulfate oder Polyoxialkylenalkylsulfaten (Alkylethersulfate) als Lösemittel bzw. Dispergiermittel eingearbeitet werden. Meist werden hierbei sehr hoch viskose, bei tieferen Temperaturen nicht mehr fliessfähige Dispersionen erhalten.

Bedingt durch den hohen Anteil von Alkylsulfaten bzw. Alkylethersulfaten, haben diese Perlglanzdispersionen folgende Nachteile:

Unverträglichkeit mit kationischen Tensiden, d.h. die vorliegenden Perlglanzkonzentrate bilden in kationischen Einstellungen, wie Haarnachspülmitteln und Wäscheweichspülmitteln, Neutralsalze, die zum Teil unlöslich sind.

Hierdurch wird die kationische Ladung geschwächt und somit der gewünschte Effekt vermindert; Hydrolyseempfindlichkeit der Alkylsulfate bzw. Alkylethersulfate, vor allem im niedrigen pH-Bereich (< pH 5); starke Schaumbildung bei der Verarbeitung durch Anwesenheit von grossen Mengen stark schäumender Tenside; hohe Viskosität bei niedrigen Temperaturen und damit erschwerte Handhabung bei Transport und Verarbeitung; Formulierung von alkylsulfat- bzw. alkylethersulfatfreien Einstellungen nicht möglich; Anwesenheit von toxikologisch bedenklichem Dioxan bei Verwendung von alkylethersulfathaltigen Perlglanzkonzentraten.

Um die obengenannten Nachteile der auf dem Markt befindlichen Perlglanzdispersionen zu eliminieren, wurden umfangreiche Untersuchungen durchgeführt. Es wurde überraschend gefunden, dass mit Fettsäuremono- und/oder Polyglykolestern in Kombination mit Fettsäurealkanolamiden Perlglanzdispersionen erhalten werden, die neben einem ausgezeichneten Perlglanzeffekt und einer guten Lagerstabilität die obengenannten Nachteile nicht aufweisen, wenn man als Netz- bzw. Dispergiermittel ein nichtionisches Tensid verwendet, wie weiter unten definiert.

Gegenstand der Erfindung sind nichtionische, fliessfähige Perlglanzdispersionen im wesentlichen bestehend aus

5-30 Gew.-% eines Fettsäureglykolesters der allgemeinen Formel I

$$R_1-\overset{\overset{\displaystyle O}{\|}}{C}-(O-A)_m-O-X \qquad (I)$$

wobei $R_1$ eine lineare oder verzweigte, gesättigte oder ungesättigte Kohlenwasserstoffkette mit 13-21 Kohlenstoffatomen, A eine Gruppe der Formel $-C_2H_4-$ oder $-C_3H_6-$, vorzugsweise $-C_2H_4-$, x ein Wasserstoffatom oder eine Gruppe der allgemeinen Formel

$$R_1-\overset{\overset{\displaystyle O}{\|}}{C}-$$

und m eine Zahl von 1-10, vorzugsweise 1-3 bedeuten,

2-20 Gew.-% eines Fettsäurealkanolamides der allgemeinen Formel II

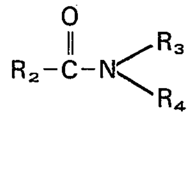

(II)

wobei $R_2$ eine lineare oder verzweigte, gesättigte oder ungesättigte Kohlenwasserstoffkette mit 7-29 Kohlenstoffatomen,

$R_3$ und $R_4$ unabhängig voneinander ein Wasserstoffatom oder eine Gruppe der Formel $-C_2H_4OH$ oder $-C_3H_6OH$ bedeuten,

0,1-10 Gew.-% eines nichtionischen Tensides der allgemeinen Formel III

$$R_5-O-(A-O)_m-(B-O)_n-R_6 \qquad (III)$$

wobei $R_5$ eine lineare oder verzweigte, gesättigte oder ungesättigte Kohlenwasserstoffgruppe mit 8-30, vorzugsweise 10-18 Kohlenstoffatomen, $R_6$ ein Wasserstoffatom oder eine Gruppe der allgemeinen Formel

$$-CH_2-O-R_7$$

$R_7$ eine lineare oder verzweigte, gesättigte oder ungesättigte Kohlenwasserstoffgruppe mit 1-10, vorzugsweise 3-5 Kohlenstoffatomen, A und B unabhängig voneinander eine Gruppe der Formel $-C_2H_4-$ oder $-C_3H_6-$ und n und m jeweils für sich eine Zahl von 1-30, vorzugsweise 3-10 bedeuten, sowie entsalztes Wasser in der an 100% fehlenden Menge.

Im Rahmen der obengenannten Einsatzkonzentrationen der vier Komponenten werden unter Einhaltung der nachstehend beschriebenen Bedingungen optimale Perlglanzdispersionen erhalten, die gegenüber den z.Z. auf dem Markt angeboten Perlglanzdispersionen folgende Vorteile aufweisen:

a) Nichtionischer Aufbau, dadurch keine chemische Reaktion mit kationischen Tensiden.

b) Ausserordentlich günstiges Fliessverhalten auch bei tieferen Temperaturen.

c) Sehr niedriger Tensidanteil.

d) Frei von Alkylsulfaten bzw. Alkylethersulfaten.

e) Weitgehende Erniedrigung des Dioxangehaltes.

Die zur Herstellung der erfindungsgemässen Perlglanzdispersionen mit optimalen anwendungstechnischen Eigenschaften benötigten Komponenten sowie die Einsatzkonzentrationen werden nachfolgend beschrieben.

Als Perlglanzbildner haben sich Fettsäureglykolester der allgemeinen Formel (I) sowie Mischungen dieser Substanzklasse bewährt. Die günstigsten Eigenschaften zeigen Verbindungen, in denen $R_1$ ein Alkylrest mit 15-17 Kohlenstoffatomen, m=1 und x ein Rest der Formel $R_1-CO-$ ist. Die Einsatzkonzentration dieser Komponenten beträgt vorzugsweise 10-20 Gew.-%, wobei die optimale Konzentration mit 16% ermittelt wurde.

Unter den Fettsäurealkanolamiden gemäss der allgemeinen Formel (II) werden vorzugsweise solche verwendet, die in der Ausgangsfettsäure eine Verteilung von 10-18 Kohlenstoffatomen besitzen. Bevorzugt werden hierbei Fettsäuremonoethanolamide verwendet, wobei einer der beiden Reste $R_3$ oder $R_4$ der allgemeinen Formel (II) ein Wasserstoffatom und der andere Rest eine Gruppe der Formel $-C_2H_4OH$ darstellt.

Die Einsatzkonzentration dieser Komponente beträgt vorzugsweise 2-10%, insbesondere 4%.

Unter den geeigneten nichtionischen Tensiden entsprechend der allgemeinen Formel III haben sich speziell solche Verbindungen als günstig erwiesen, bei denen $R_5$ eine Alkylgruppe mit 10-18 Kohlenstoffatomen, linear oder verzweigt und m bzw. n jeweils 4 darstellen. Die Einsatzkonzentration dieser nichtionischen Komponente beträgt vorzugsweise 0,5-5%, insbesondere 2%. Bei dem in der vorliegenden Erfindung verwendeten Wasser handelt es sich um entsalztes Wasser.

Zur Herstellung der erfindungsgemässen Perlglanzdispersion wird wie folgt verfahren. In einem Mischgefäss (heizbarer Kessel) werden die beiden Komponenten der allgemeinen Formel I und II vorgelegt und unter Erhitzen auf ca. 76° C geschmolzen. In dieses Schmelze wird unter Rühren eine ebenfalls auf 75° C erhitzte wässerige Lösung der Komponente der allgemeinen Formel III zugegeben. Die Rührgeschwindigkeit sollte nicht zu hoch gewählt werden und in etwa zwischen 10-100 Umdrehungen pro Minute betragen. Die gebildete Dispersion wird bei einer Temperatur von 75° C noch ca. 30 Minuten gerührt. Anschliessend wird unter Rühren bis zu einer Endtemperatur von 20-30° C abgekühlt. Die Geschwindigkeit der Abkühlung sollte unter kontrollierten Bedigungen erfolgen nämlich in der gleichen Weise, wie es auch bei den bisher gebräuchlichen Perlglanzdispersionen gemacht wird, um einen konstanten, reproduzierbaren Perlglanzeffekt zu erreichen.

Während des Abkühlvorgangs beginnen die beiden Komponenten I und II bei ca. 50-60° C zu kristallisieren und bilden den gewünschten Perlglanz.

Neben den genannten Hauptkomponenten kann die erfindungsgemässe Perlglanzdispersion noch Zusätze wie Konservierungsmittel, Puffersubstanzen sowie Elektrolyte enthalten. Der pH-Wert der Dispersion liegt vorzugsweise im Bereich von 4-9, vorzugsweise bei 5-8.

Die vorliegende, erfindungsgemässe Perlglanzdispersion kann bei Raumtemperatur flüssigen Haar- und Körperreinigungsmitteln, flüssigen Geschirrspülmitteln sowie flüssigen Wasch- und Reinigungsmitteln zugesetzt werden. Hierdurch werden Endprodukte erhalten, die einen ausgezeichneten Perlglanz aufweisen. Die hierzu benötigte Menge der Perlglanzdispersion liegt im allgemeinen zwischen 1 und 10%.

Da die erfindungsgemässe Perlglanzdispersion bei Temperaturen über 10° C eine vergleichsweise niedrige Viskosität aufweist, besteht die Möglichkeit, die Dispersion mit Hilfe von automatischen Pump-, Dosier- und Mischanlagen zu verarbeiten. Dies ist von besonderem Interesse bei der voll kontinuierlichen Herstellung von perlglanzhaltigen Fertigprodukten.

Erfindungsgemässe Perlglanzdispersionen sind in den folgenden Beispielen beschrieben. Die Mengenangaben beziehen sich jeweils auf Gewichtsprozente. «EO» bedeutet Ethylenoxid, «PO» Propylenoxid.

*Beispiel 1:*

*Zusammensetzung*

| | |
|---|---|
| Monoethylenglykoldistearat | 16,0% |
| Cocosfettsäuremonoethanolamid | 4,0% |
| $C_{10/12}$-Fettalkohol + 4 EO + 4 PO | 2,0% |
| Wasser, Konservierungsmittel | ad 100,0% |

*Beispiel 2:*

*Zusammensetzung*

| | |
|---|---|
| Monoethylenglykoldistearat | 10,0% |
| Cocosfettsäuremonoethanolamid | 10,0% |
| $C_{10/12}$-Fettalkohol + 4 EO + 4 PO | 2,0% |
| Wasser, Konservierungsmittel | ad 100,0% |

*Beispiel 3:*

*Zusammensetzung*

| | |
|---|---|
| Triethylenglykoldistearat | 16,0% |
| Cocosfettsäuremonoethanolamid | 4,0% |
| $C_{10/12}$-Fettalkohol + 4 EO + 4 PO | 2,0% |
| Wasser, Konservierungsmittel | ad 100,0% |

*Beispiel 4:*

*Zusammensetzung*

| | |
|---|---|
| Triethylenglykoldistearat | 10,0% |
| Cocosfettsäuremonoethanolamid | 10,0% |
| $C_{10/12}$-Fettalkohol + 4 EO + 4 PO | 2,0% |
| Wasser, Konservierungsmittel | ad 100,0% |

Diese Perlglanzdispersionen gemäss den Beispielen 1-4 wurden nach folgenden Kriterien geprüft:

1. Beurteilung des Perlglanzeffektes
2. Bestimmung der «optischen Dichte»
3. Viskosität bzw. Fliessverhalten
4. Lagerstabilität bei höheren und tieferen Temperaturen als Raumtemperatur.

Als Prüfmethoden wurden die üblicherweise in der Kosmetikbranche verwendeten Testmethoden herangezogen. Die Beurteilung des Perlglanzbildes erfolgte visuell im Vergleich zu den auf dem Markt befindlichen Perlglanzdispersionen. Die Beurteilung der optischen Dichte erfolgte photometrisch in einer Verdünnung von 0,5 g/l Wasser mit Hilfe eines Trübungsmessgerätes (nach Dr. Lange).

Nach diesen Prüfkriterien zeigen die in den Beispielen 1-4 beschriebenen Perlglanzdispersionen ausgezeichnete, mit handelsüblichen Produkten vergleichbare oder in besonderen Fällen günstigere Eigenschaften. Gegenüber diesen handelsüblichen Dispersionen sind die erfindungsgemässen Dispersionen aber bereits unter 20°C fliessfähig.

## Patentansprüche

1. Nichtionische, fliessfähige Perlglanzdispersionen, dadurch gekennzeichnet, dass sie im wesentlichen bestehen aus 5-30 Gew.-% eines Fettsäureglykolesters der allgemeinen Formel I

$$R_1-\overset{\overset{\displaystyle O}{\|}}{C}-(O-A)_m-O-X \qquad (I)$$

wobei $R_1$ eine gesättigte oder ungesättigte Kohlenwasserstoffkette mit 13-21 Kohlenstoffatomen,

A eine Gruppe der Formel $-C_2H_4-$ oder $-C_3H_6-$, vorzugsweise $-C_2H_4-$,

x ein Wasserstoffatom oder eine Gruppe der allgemeinen Formel

$$R_1-\overset{\overset{\displaystyle O}{\|}}{C}-$$

und m eine Zahl von 1-10, vorzugsweise 1-3 bedeuten,

2-20 Gew.-% eines Fettsäurealkanolamides der allgemeinen Formel II

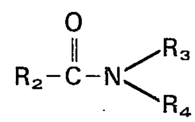

(II)

wobei $R_2$ eine gesättigte oder ungesättigte Kohlenwasserstoffkette mit 7-29 Kohlenstoffatomen, $R_3$ und $R_4$ unabhängig voneinander ein Wasserstoffatom oder eine Gruppe der Formel $-C_2H_4OH$ oder $-C_3H_6OH$ bedeuten, 0,1-10 Gew.-% eines nichtionischen Tensides der allgemeinen Formel III

$$R_5-O-(A-O)_m-(B-O)_n-R_6 \qquad (III)$$

wobei $R_5$ eine gesättigte oder ungesättigte Kohlenwasserstoffgruppe mit 8-30 Kohlenstoffatomen, $R_6$ ein Wasserstoffatom oder eine Gruppe der allgemeinen Formel

$$-CH_2-O-R_7,$$

$R_7$ eine gesättigte oder ungesättigte Kohlenswasserstoffgruppe mit 1-10 Kohlenstoffatomen, A und B unabhängig voneinander eine Gruppe der Formel $-C_2H_4-$ oder $-C_3H_6-$ und n und m jeweils für sich eine Zahl von 1-30, vorzugsweise 3-10 bedeuten, sowie entsalztes Wasser in der an 100% fehlenden Menge.

2. Perlglanzdispersionen nach Anspruch 1, dadurch gekennzeichnet, dass in der Formel I $R_1$ $C_{15}-C_{17}$-Alkyl, X eine Gruppe der Formel $R_1CO-$ und m 1 bedeuten, in der Formel II $R_2$ $C_9-C_{17}$-Alkyl, $R_3$ Wasserstoff und $R_4$ die Gruppe $-C_2H_4OH$ bedeuten und in der Formel III $R_5$ $C_{10}-C_{18}$-Alkyl, $R_7$ $C_3-C_5$-Alkyl und m und n jeweils 4 bedeuten.

3. Perglanzdispersionen nach Anspruch 1, dadurch gekennzeichnet, dass sie 10-20 Gew.-% der Komponente I, 2-10 Gew.-% der Komponente II und 0,5-5 Gew.-% der Komponente III enthält.

## Claims

1. A non-ionic fluent pearl luster dispersion essentially comprising 5-30% by weight of a fatty acid glycol ester of the formula I

$$R_1-\overset{\overset{\displaystyle O}{\|}}{C}-(O-A)_m-O-X \qquad (I)$$

where $R_1$ denotes a saturated or unsaturated hydrocarbon chain of 13-21 carbon atoms, A denotes a group of the formula $-C_2H_4-$ or $-C_3H_6-$, preferably $-C_2H_4-$, x denotes a hydrogen atom or a group of the formula

$$R_1-\overset{\displaystyle O}{\overset{\|}{C}}-$$

and m denotes a number from 1 to 10, preferably 1 to 3, 2-20% by weight of a fatty acid alkanolamide of the formula II

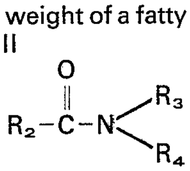 (II)

where $R_2$ denotes a saturated or unsaturated hydrocarbon chain of 7-29 carbon atoms, and $R_3$ and $R_4$, independently of each other, denote a hydrogen atom or a group of the formula $-C_2H_4OH$ or $-C_3H_6OH$, 0,1-10% by weight of a non-ionic surfactant of the formula III

$$R_5-O-(A-O)_m-(B-O)_n-R_6 \qquad (III)$$

where $R_5$ denotes a saturated or unsaturated hydrocarbon group of 8-30 carbon atoms, $R_6$ denotes a hydrogen atom or a group of the formula

$$-CH_2-O-R_7,$$

$R_7$ denotes a saturated or unsaturated hydrocarbon group of 1-10 carbon atoms, A and B, independently of each other, denote a group of the formula $-C_2H_4$ or $-C_3H_6-$, and n and m each denote a number from 1 to 30, preferably 3 to 10, and demineralized water to 100%.

2. A pearl luster dispersion as claimed in claim 1, wherein, in the formula I, $R_1$ denotes $C_{15}$-$C_{17}$-alkyl, X denotes a group of the formula $R_1CO-$ and m denotes 1, in the formula II $R_2$ denotes $C_9$-$C_{17}$-alkyl, $R_3$ denotes hydrogen and $R_4$ denotes a $C_2H_4OH$ group, and in the formula III $R_5$ denotes $C_{10}$-$C_{18}$-alkyl, $R_7$ denotes $C_3$-$C_5$-alkyl and m and n each denote 4.

3. A pearl luster dispersion as claimed in claim 1 which contains 10-20% by weight of component I, 2-10% by weight of component II and 0.5-5% weight of component III.

## Revendications

1. Dispersions nacrées fluides, non ioniques, caractérisées en ce qu'elles sont essentiellement constituées de 5-30% en poids d'un ester d'acide gras et d'un glycol, de formule générale I

$$R_1-\overset{\displaystyle O}{\overset{\|}{C}}-(O-A)_m-O-X \qquad (I)$$

dans laquelle $R_1$ est une chaîne hydrocarbonée saturée ou insaturée ayant de 13-21 atomes de carbone,

A est un groupe de formule $-C_2H_4-$ ou $-C_3H_6-$, de préférence $-C_2H_4-$,

x est un atome d'hydrogène ou un groupe de formule générale

$$R_1-\overset{\displaystyle O}{\overset{\|}{C}}-$$

et m est un nombre de 1-10, de préférence de 1-3,

2-20% en poids d'un alcanolamide d'acide gras, de formule générale II

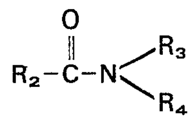 (II)

dans laquelle $R_2$ est une chaîne hydrocarbonée, saturée ou insaturée, ayant de 7-29 atomes de carbone,

$R_3$ et $R_4$, indépendamment l'un de l'autre, sont chacun un atome d'hydrogène ou un groupe de formule $-C_2H_4OH$ ou $-C_3H_6OH$,

0,1-10% en poids d'un surfactif non ionique de formule générale III

$$R_5-O-(A-O)_m-(B-O)_n-R_6 \qquad (III)$$

dans laquelle $R_5$ est un groupe hydrocarboné saturé ou insaturé ayant de 8-30 atomes de carbone,

$R_6$ est un atome d'hydrogène ou un groupe de formule

$$-CH_2-O-R_7,$$

$R_7$ est un groupe hydrocarboné saturé ou insaturé ayant de 1-10 atomes de carbone,

A et B, indépendamment l'un de l'autre, sont chacun un groupe de formule $-C_2H_4-$ ou $C_3H_6-$, et n et m sont, chacun indépendamment de l'autre, un nombre compris entre 1-30, de préférence entre 3-10,

ainsi que d'eau déminéralisée, en la quantité permettant le complément à 100%.

2. Dispersions nacrées selon la revendication 1, caractérisées en ce que, dans la formule I, $R_1$ désigne un groupe alkyle en $C_{15}$-$C_{17}$, X est un groupe de formule $R_1CO-$ et m vaut 1; dans la formule II, $R_2$ désigne un groupe alkyle en $C_9$-$C_{17}$, $R_3$ est l'hydrogène et $R_4$ est le groupe $-C_2H_4OH$; et dans la formule III, $R_5$ est un groupe alkyle en $C_{10}-C_{18}$, $R_7$ est un groupe alkyle en $C_3$-$C_5$, et m et n valent chacun 4.

3. Dispersions nacrées selon la revendication 1, caractérisées en ce qu'elles contiennent de 10-20% en poids du constituant I, de 2-10% en poids du constituant II et de 0,5-5% en poids du constituant III.